Europäisches Patentamt

European Patent Office

Office européen des brevets

(11) Publication number: **0 028 162**
**B1**

# EUROPEAN PATENT SPECIFICATION

(45) Date of publication of patent specification: **23.01.85**

(51) Int. Cl.⁴: **A 61 M 15/00**

(21) Application number: **80303872.8**

(22) Date of filing: **30.10.80**

(54) **Breath actuated devices for administering powdered medicaments.**

(30) Priority: **30.10.79 GB 7937519**

(43) Date of publication of application:
**06.05.81 Bulletin 81/18**

(45) Publication of the grant of the patent:
**23.01.85 Bulletin 85/04**

(84) Designated Contracting States:
**FR IT**

(56) References cited:
**FR-A-2 380 032**
**FR-A-2 388 566**
**GB-A-1 520 064**

(73) Proprietor: **RIKER LABORATORIES, INC.**
**19901 Nordhoff Street**
**Northridge California (US)**

(72) Inventor: **Baum, Eric Arthur**
**Minnesota 3M Research Limited Pinnacles**
**Harlow Essex (GB)**
Inventor: **Davies, Leslie James**
**Minnesota 3M Research Limited Pinnacles**
**Harlow Essex (GB)**
Inventor: **Kirk, William Francis**
**Riker Laboratories 1 Morley Street**
**Loughborough Leicestershire (GB)**
Inventor: **Wass, Anthony Charles Lammon**
**The Mousehole Duddington**
**Stamford Lincolnshire (GB)**

(74) Representative: **Bowman, Paul Alan et al**
**LLOYD WISE, TREGEAR & CO. Norman House**
**105-109 Strand**
**London WC2R OAE (GB)**

Courier Press, Leamington Spa, England.

## Description

This invention relates to a device for administering powdered medicaments by oral inhalation and which is actuated by the inhalation of the patient.

There are many devices for administering powdered medicaments to the alveolar region of the lungs which employ propellants, such as compressed gases, e.g. air, or liquefied gas propellants, to dispense and disperse the medicament. These devices tend to be complex to construct and have the disadvantage that in order for the optimum effect to be obtained it is essential that inhalation and dispensing be synchronous.

There are also a number of known breath actuated inhalation devices for administering powdered medicaments to the lungs which have mouthpieces through which the medicament is inhaled. The powdered medicament is usually supplied in capsules which are substantially cylindrical in shape and have rounded ends. The capsule is formed in two halves having different diameters, one half being a push-fit within the other. In the simpler breath actuated devices the capsule is opened prior to insertion into the device and one half of the capsule containing the medicament or the medicament itself is positioned in the device such that during inhalation through the mouthpiece the medicament becomes entrained in the air stream and passes to the patient. Examples of such devices are disclosed in British Patent Specification Nos. 1 520 064, 1 504 441, 1 118 341, 1 520 063 and 1 520 062. These devices suffer from the disadvantage that medicament may be spilled when the capsule is opened prior to insertion in the device.

British Patent Specification Nos. 1 521 000, 1 472 650 and 1 502 150 disclose more complex devices in which the complete capsule is inserted into the device thus ensuring no spillage of medicament prior to inhalation, and access to the medicament is gained by piercing the capsule or cutting it in half, inside the dispensing device. On inhalation the air flows into or through the capsule and the powder within is released into the air stream and flows towards the mouth.

The devices disclosed in British Patent Specification Nos. 1 485 163, 1 331 216, 1 457 352, 1 396 258, 1 182 779, 1 404 338, 1 459 426 and 1 118 341 and United States Patent Specification Nos. 4 117 844 and 4 116 195 are designed to agitate the capsule containing the medicament in order to help release and disperse the powder from the capsule. The capsule may be agitated by various means, e.g. creating a turbulent air flow, or by rotational and/or vibrational motion of the capsule with the use of vanes or propellors. These devices incorporate complex arrangements and are often difficult to operate.

FR—A—2 380 032 discloses a device in which access to the powdered medicament is gained by pulling the halves of the capsule apart so that the medicament is emptied to a suitable position for entrainment in the air flow caused by inhalation.

In spite of the numerous prior art devices there is a need for a breath actuated device which is simple to operate and efficient in administering powdered medicaments into the alveolar region of the lungs.

The present invention provides a breath actuated device for oral inhalation of medicament in finely divided powder form in a capsule comprising a base component and a lid component, the base component being capable of holding said powdered medicament and the lid component being capable of being removed to expose the powdered medicament in the base component, said device comprising a chamber having one or more inlet ports for entry of air and an outlet port in direct communication with a mouthpiece and, positioned within the chamber, the base component of a capsule containing said medicament, characterised in that the base component of the capsule is mounted substantially vertically and has a substantially horizontal rim defining an aperture and that the inlet ports and outlet port are arranged such that when the device is operated by suction at the mouthpiece a substantially horizontal air flow is established from the inlet ports to the outlet port directly across and substantially parallel to said rim causing particles of medicament to become entrained in said air flow.

In a preferred embodiment, there is positioned within the chamber, means for separating the lid and base components of a capsule containing the medicament whilst holding the capsule substantially vertically to expose an aperture in the base component facilitating access to the powdered medicament.

In the operation of the devices of the invention it is the base component of the capsule which contains the powdered medicament, the lid component having been removed.

Whilst the invention is described mainly with reference to conventional capsules used in the pharmaceutical industry which are substantially cylindrical and are formed of two halves, one being a force fit within the other, the invention is equally applicable to other containers in which access to the contents is gained by removal of a lid component, e.g. a tube having a bung or a container having a foil lid adhered or crimped over the open end. The devices of the invention may include the base component of the capsule integrally mounted therein as will be described hereinafter.

The device according to the invention is simple to operate and is effective in transferring medicaments to the lungs. The complete capsule containing medicament is placed in the device thereby preventing spillage of the medicament and the lid and base components of the capsules are separated, e.g. by pulling apart within the device to allow direct and efficient access to the powder held in the base component without need for the operator to manually handle the capsule during the opening operation. A mouthpiece is arranged with direct access to the chamber in which the base component of the capsule containing the medicament is held in order that the medicament may have a short direct path to the mouth. Constructions, corners or obstructions would hinder the passage of powder. The inlet port or ports to the chamber, which may be in the form of a slit or a plurality of radial apertures, are arranged to provide an air flow passing directly over the aperture in the base of the capsule containing medicament so that the powder contained in the capsule base component will be entrained into the air stream.

The airflow established in the device of the invention is directed uniformly across the open aperture and not into the base component of the capsule. This airflow is effective in entraining powder from the capsule possibly due to a resonance effect built up in the capsule (in a similar manner to blowing over the top of an open bottle). Tests which have been conducted reveal that for optimum efficiency the airflow must be across the open aperture and there tends to be an optimum position for the medicament and hence the capsule relative to the lower extent of the inlet ports, i.e. relative to the airflow. This optimum position may vary according to capsule dimensions and the amount of medicament in the capsule but does not tend to be critical for capsule bases which are relatively shallow, e.g. up to 10 mm, but becomes more critical when deeper capsule bases are used. In general it has been found that the rim of the capsule should not extend significantly above the lower extent of the inlet ports and is preferably at the same level or slightly lower than the lower extent of the inlet ports, i.e. the medicament in the capsule base is spaced slightly from the mainstream of the airflow. Preferably, the device of the invention is arranged to take a particular size of base component of a capsule and includes means to accurately position the capsule base in the optimum position for powder entrainment in the airflow.

The efficiency achieved by using an airflow directly across the aperture of the base of the capsule is surprising since heretofore it was always believed that the airflow in such devices should impinge directly onto the powder and devices of the prior art were specifically designed to create sufficient turbulence in order to entrain the powder in the airflow. For example, the device disclosed in United States Patent Specification No. 4 192 309 which utilizes a vertically positioned opened capsule as a powder reservoir deliberately incorporates a deflector to deflect the air stream into the opened capsule to entrain the powder. Tests conducted on the device in accordance with the invention have shown that the inclusion of a deflector to deflect air into the capsule does not affect the efficiency of the device.

The inlet and outlet ports of the chamber are arranged so that the airflow from the former to the latter passes directly over the opened capsule base. The lower extent of the inlet port is preferably at the same height or higher than the lower extent of the outlet port and the vertical extent of the aperture of the inlet ports is wholly within that of the outlet port so that in use a substantially horizontal flow of air passes from inlet to outlet across the opened capsule base. The total cross-sectional area of the inlet ports is small compared with that of the outlet port so that the velocity of the air across the capsule base is high. However, the pressure drop across the chamber in use should not be high since this would require the user to exert considerable suction at a time when he is in a distressed condition. In practice it has been found that it is possible to achieve a suitable arrangement of inlet and outlet ports which will give the desired airflow and will cause a pressure drop of about 490 Pa (5 cm of water) which is barely noticeable to the user. A pressure drop of up to 2452 Pa (25 cm of water) appears to be an upper limit as far as comfort to the user is concerned.

The mouthpiece is preferably short and straight and aligned with the airflow across the chamber so that there is a substantially straight airflow across the device. However, the mouthpiece may be shaped or inclined slightly with respect to the airflow across the chamber without significantly affecting the efficiency of the device providing there are no obstructions which will trap the particles entrained in the airflow. The mouthpiece of the device may be adapted for nasel inhalation and the term "mouthpiece" used herein includes such nasal adaptors.

According to one embodiment the capsule base component containing the medicament is supported vertically on a sliding piston arrangement so that when the device is operated by suction the piston is urged upwards by reduced pressure until it abuts a stop; this action gives the powder an initial impetus which aids in the dispersion of the powder as the air stream flows across the open end of the capsule. This embodiment is particularly effective when the powder in the capsule has become compacted although the piston arrangement is not necessary for free flowing powders.

The means for separating the two components of the capsule may comprise an opening means movable between an engaging position in which the opening means engages the lid component of a capsule held by the base component in the securing means, and a dispensing position whereby movement of the opening means from its engaging position to its dispensing position causes separation of the two components of the capsule.

According to one embodiment of the invention the chamber is substantially cylindrical in shape and the device is hinged to give access to the chamber for insertion of the capsule. The roof of the chamber is provided with a clutch into which the capsule is inserted, the clutch gripping the lid component of the capsule. The base of the chamber is similarly provided with a clutch mechanism and by relative movement of the roof and the base of the chamber towards each other, the base component

3

# O 028 162

of the capsule may be engaged in the clutch at the base. Relative movement of the roof and the base of the chamber away from each other separates the two components of the capsule, the base component of the capsule holding the medicament being supported vertically by the clutch of the base. The mouthpiece is provided in one side of the chamber and a number of radial inlet ports are provided which are coplanar and are disposed over not more than 180°. The axis of the mouthpiece preferably bisects the angular spread of the inlet ports, the lower extent of the inlet ports being higher than the lower extent of the outlet port, the vertical extent of the inlet ports being wholly within that of the outlet port. The top of the base component of the capsule is arranged at substantially the same height or below the lower extent of the inlet ports. The base of the chamber may be a movable piston arrangement as described above.

In a preferred aspect, the invention also provides a device including a conveyor containing a plurality of capsules which may be introduced into the chamber in turn, opened and the medicament dispensed thereby allowing several doses to be sequentially inhaled without recharging the device. In a preferred embodiment the device comprises a carousel which retains a plurality of capsules by their base components (or includes the base components integrally moulded therein) and may be rotated to introduce the capsules sequentially into the chamber. The carousel may be moulded from plastics material and may include partitions or ribs to define one or more walls and the floor of the chamber around each capsule.

The device may include a body portion having the mouthpiece integral therewith, the body containing and supporting a carousel. A lid may be provided hinged to the body, the lid covering the top of the carousel, the opening means and the mouthpiece. In a preferred embodiment the device is constructed so that when the lid is closed the opening means is in the engaging position and when the lid is raised the opening means moves to its dispensing position thereby opening the capsule in the chamber. This is particularly desirable when the device is used by asthmatic patients since the operations required by the patient to gain relief during an asthmatic attack are minimal, the lid of the device is simply raised and the medicament inhaled.

The invention will now be described with reference to the accompanying drawings, in which:

Figure 1 represents a longitudinal section through a device in accordance with the invention,

Figure 2 is a cross-section along the line X—X of Figure 1,

Figure 3 is a diagram showing the construction of a conventional capsule for powdered medicament,

Figures 4 and 4a are longitudinal sections through modified versions of the device shown in Figure 1,

Figures 5 to 7 show a further device in accordance with the invention, the views being respectively an exploded view, a section with lid closed and a section in use,

Figures 8 to 10 show a further device in accordance with the invention, the views being respectively an exploded view, a section during the loading operation and a section in use,

Figures 11 to 13 show a plan view and sections with the lid open and closed respectively of a further device in accordance with the invention, and

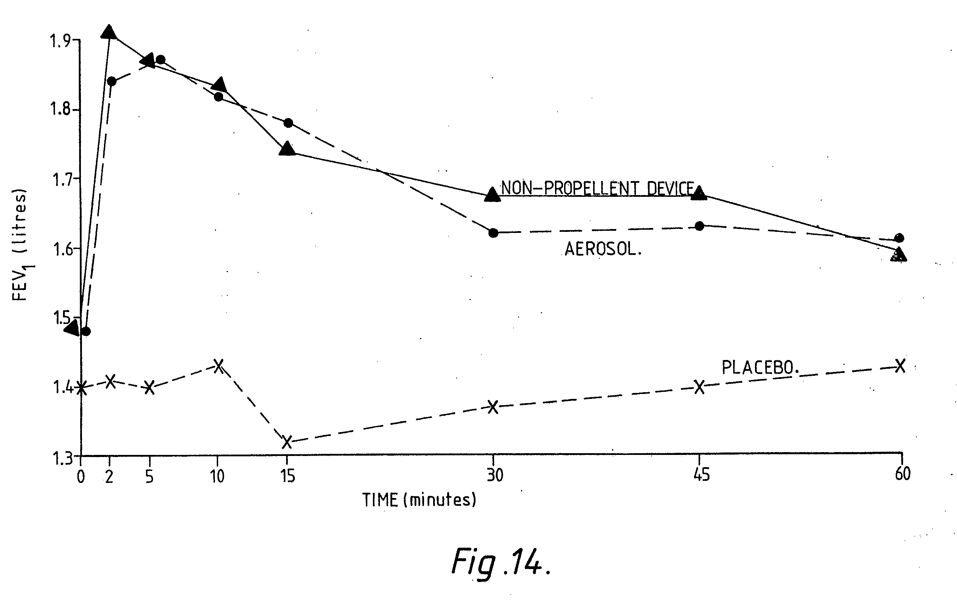

Figure 14 is a plot of ($FEV_1$) against time summarising results of a clinical trial reported hereinafter.

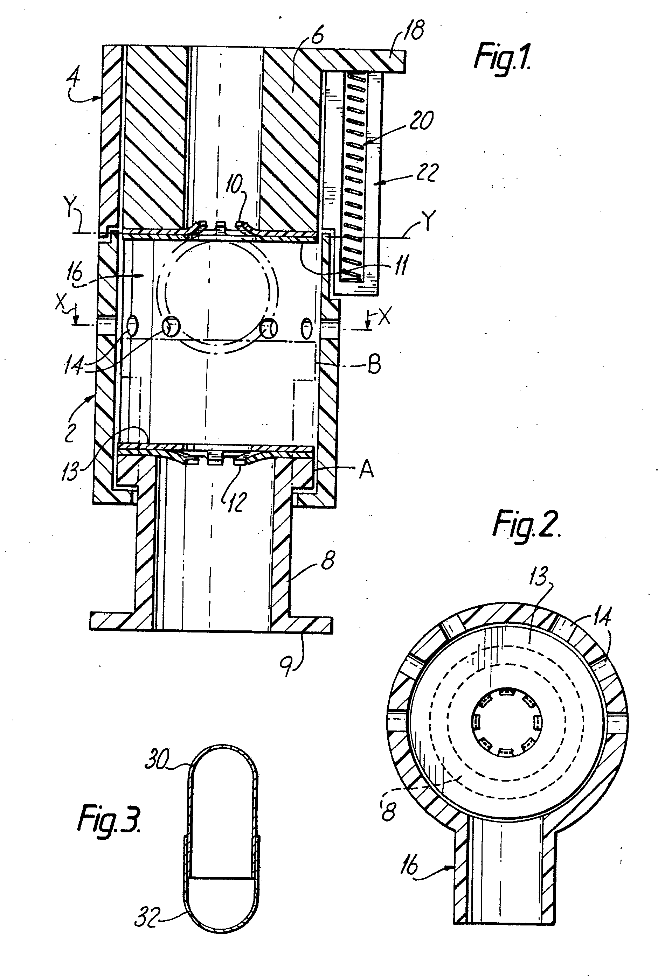

The inhalation device shown in Figures 1 and 2 comprises a cylindrical chamber defined by two cylindrical members 2 and 4 which are joined by means of a hinge at Y—Y. Within the cylindrical chamber are two pistons 6 and 8, the lower piston 8 being free to move between two extreme positions designated in Figure 1 by the lower position A and the higher position B shown in dashed outline. The limit of upward movement of the piston 8 is governed by an annular rim 9 which abuts the lower end of the casing of the cylindrical member 2.

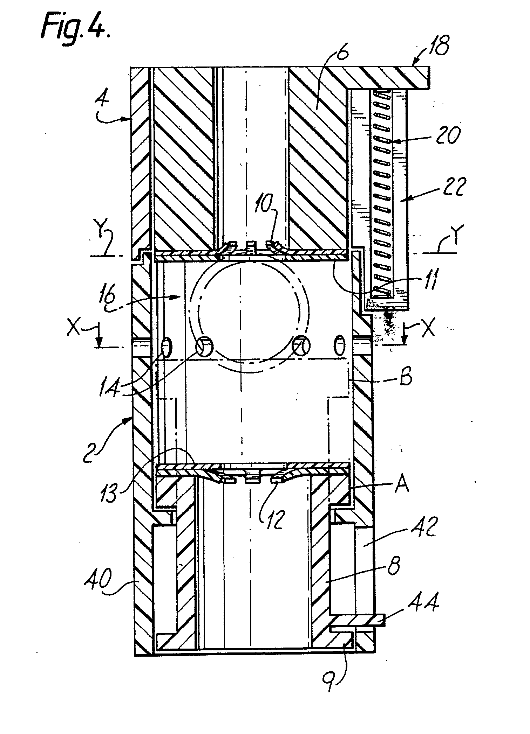

The upper piston 6 may be lowered to touch the lower piston 8 when in position B by depressing lever 18 which acts against spring 20 positioned within housing 22. The spring 20 may be replaced by a concentric spring on the outside of the piston 6 or a plurality of springs disposed about the circumference of the piston 6 in a similar manner to spring 20. The parallel end surfaces of the pistons 6 and 8 house clutch mechanisms 10 and 12 for gripping the lid and base components of the capsule. The clutch mechanism in each piston is positioned behind a flat concentric disc 11 and 13 respectively and consists of an annulus of any thin springy material such as copper-beryllium foil or plastics, e.g. polypropylene. A number of radial slots are provided in the inner circumference of the annulus to form a ring of small flexible teeth which can grip the capsule and effectively prohibit its movement back into the chamber. The apertures and clutch arrangements in the discs 11 and 13 may conveniently be dimensioned to accommodate conventional capsules used in the pharmaceutical industry as illustrated in Figure 3. Such capsules comprise a body 30 which is a force fit within a cap 32. Such capsules may be used either way up in the devices of the invention, i.e. the cap 32 may constitute the lid or the base portion of the capsule. Preferably the powdered material is retained in the cap 32 and the body 30 removed since the position of the cap with respect to the airflow is not as critical as when the body 32 is used. In the device depicted in Figure 1 the aperture in the disc 11 is large enough to accept the body 30 of the capsule but not the cap 32 and the aperture in the disc 13 is large enough to accept the cap 32.

4

The cylindrical chamber is provided with six air inlet ports 14 which are coplanar at X—X and consist of radial apertures disposed over an arc up to 180°. The diameter of the air inlet ports may be 2 mm. A cylindrical mouthpiece 16 is provided diametrically opposite the inlet ports such that the axis of the mouthpiece bisects the angular spread of the inlet ports when viewed from above (the axes being in different planes). The lower extent of the inlet ports is higher than the lower extent of the mouthpiece but lower than the axis of the mouthpiece. When the lower piston 8 is in position B its top surface, the top of the capsule base component, and the lower extent of the inlet ports are coplanar. With this arrangement of inlet ports, the pressure drop developed across the device during normal inhalation may be kept sufficiently low as to be almost unnoticeable to the patients. For example, for a pulmonary inhalation rate of 28.3 l/min the pressure drop developed may be 4.61 Pa (equivalent to a 4.7 cm head of water). The arrangement of the inlet ports and mouthpiece may be selected to obtain the desired pressure drop which is generally maintained below 2452 Pa (25 cm of water) at an inhalation rate of 28.3 l/min.

The device is operated as follows.

The device is opened by pivoting about the hinge at Y—Y. A capsule having a body 30 and a cap 32 is inserted body first through the aperture in the disc 11 and is gripped by the clutch mechanism 10. The capsule is pushed in until the cap of the capsule abuts the surface of the disc 11. In this position the complete cap protrudes from the disc 11.

The device is then closed and with the lower piston 8 held in position B, the upper piston 6 is lowered by depressing lever 18. The cap is pushed into the aperture of disc 13 and is gripped by clutch 12. The upper piston 6 is then allowed to return to its original position under the action of the spring 20. This action pulls the two halves of the capsule apart with the powdered medicament located in the cap which is held vertically in the clutch 12. The lower piston 8 is allowed to fall to its rest position at A.

The patient inhales through the mouthpiece 16, the piston 8 is accelerated upwards to position B, air flows through the inlet ports directly across the open end of the cap and powder flows out of the cap becoming entrained in the air flow and thus passes through the mouthpiece and into the alveolar region of the lungs of the patient. The empty halves of the capsule are subsequently ejected from the clutch mechanisms of the device upon insertion of a new capsule.

The efficiency of the device in transferring powdered medicaments into the alveolar region of the lungs may be determined by measuring the particle size distribution of a micronized bronchodilating drug emitted from the device on an aerodynamic particle sampler known as the Andersen sampler Model No. 22—000 manufactured by Andersen 2000 Inc. of Atlanta, Georgia, United States of America, which is a recognised instrument for measuring airborne particle size distributions. In particular the amount of drug collected on stages 3 to 7 of the Andersen sampler which represents particles between 4.7 $\mu$m and 0.43 $\mu$m, was observed. This fraction represents the amount of drug which is small enough to be transmitted into the lungs and is known as the respirable fraction.

In one series of tests 15 mg samples of a blend of micronised isoprenaline sulphate (1.00 $\mu$g) in powdered lactose was introduced into standard size 4 capsules. See Remingtons Pharmaceutical Sciences, Mack Publishing Company, 15th Edition, 1975 pages 1598 to 9 for details of capsule sizes. A capsule was inserted cap downwards in a device of the invention. The device was connected to the Andersen sampler and air was drawn through the system at a rate of 28.3 l/min. The resultant respirable fraction calculated as a percentage of the total dose of drug originally packed in the capsule obtained with the new device is given below together with respirable fractions obtained on the Andersen sampler with two other prior art devices using the same powder formulations:

| Device | Respirable fraction |
|---|---|
| Device of the invention | 20.3% |
| British Patent No. 1 182 779 | 9.1% |
| United States Patent No. 4 117 844 | 3.9% |

These results confirm the efficiency of the device of Figure 1 of the invention over devices of the prior art.

It has been found that when capsules are used in which the powder is uncompacted and free flowing the efficiency of drug transfer into the lungs is substantially the same for the following situations:

(i) when the sliding piston 8 is allowed to slide during inhalation from position A to position B, and
(ii) when the piston is held static in position B.

However, when highly cohesive or compacted powders are present in the capsules the sliding piston arrangement (i) is considerably more efficient than the static arrangement (ii) as shown in the following table:

Powder: Compacted impalpable Lactose.
    15 mg/capsule.

|  | Sliding piston | Piston held static in position B |
|---|---|---|
| Average removed as % of total | 78% | 44% |

A further series of tests was performed to examine the efficiency of the airflow directly across the open capsule portion in the device of the invention compared with an airflow deflected into the capsule portion.

A deflector to channel the inspired air into the capsule containing the drug blend was built into a device of the invention with the clutch holder static in position B. A determination was made by an Andersen Sampler of the respirable fraction obtained by using this modified device with a blend of micronised rimiterol hydrobromide 0.25 mg with lactose 45 mg. The respirable fraction obtained from the same blend using a similar device without a deflector was also determined. The results are shown in the following table.

| Drug found as % of total drug filled into capsules | No deflector | | Deflector | |
|---|---|---|---|---|
|  | 1 | 2 | 1 | 2 |
| Capsules and device | 12.4 | 11.1 | 13.1 | 15.7 |
| Throat and pharnyx of Andersen | 53.7 | 57.6 | 52.1 | 55.0 |
| Upper Andersen | 17.8 | 16.5 | 18.4 | 14.6 |
| Andersen plates 3 to 7 (respirable fraction) | 16.1 | 14.8 | 16.3 | 14.7 |

The results indicate that inclusion of a deflector did not increase the respirable fraction.

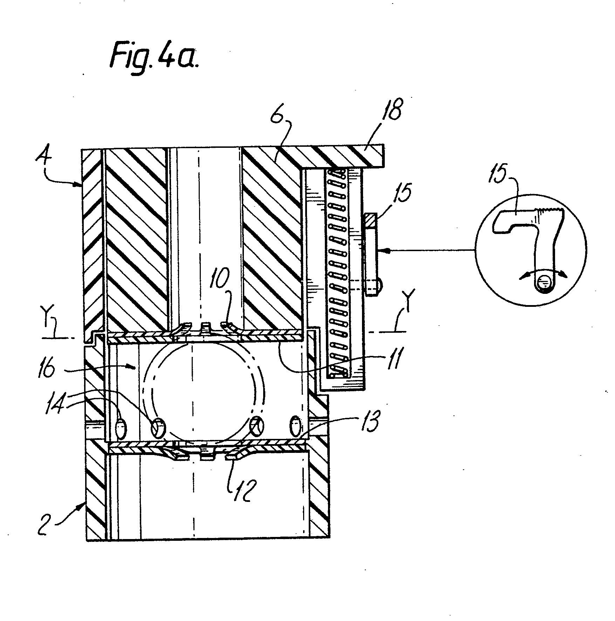

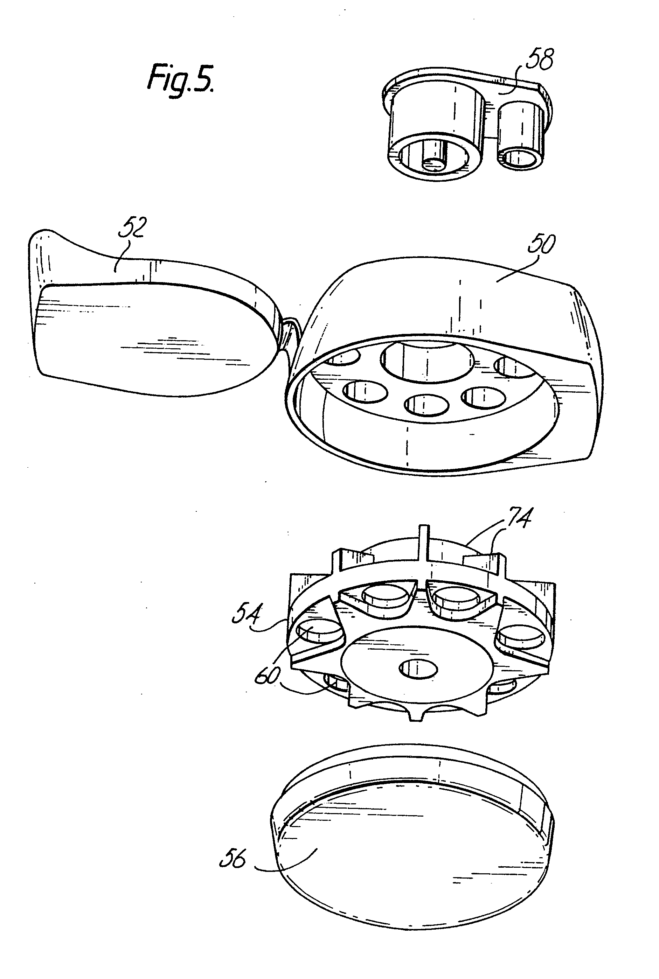

Detailed tests were undertaken to compare the efficiency of a device of the invention with that illustrated in Figures 4 and 5 of FR—A—2380032.

In the device of the prior art an opened capsule has its long axis horizontal and parallel to the mouthpiece. The unopened capsule is fitted into clutch mechanisms one of which is movable to pull the capsule apart. In practice difficulty was experienced in separating the capsule halves.

The respirable fraction was determined upon size 4 capsules containing a blend of micronised rimiterol hydrobromide 0.25 mg and lactose 45 mg as in the preceding tests. Four determinations were performed according to the position of the powder:

A.  divided approximately equally between the two capsule halves,
B.  all in the capsule half with its open end facing away from the mouthpiece,
C.  all in the capsule half with its open end facing the mouthpiece, and
D.  deliberately tipped onto the floor of the chamber.

The results are shown in the following table.

| Locus of Drug (% of total drug filled into capsules) | A | B | C | D |
|---|---|---|---|---|
| Capsule and device | 91.6 | 92.3 | 91.7 | 23.2 |
| Throat and pharynx | 3.2 | 3.3 | 2.5 | 51.8 |
| Upper Andersen | 2.9 | 2.4 | 2.9 | 16.7 |
| Respirable fraction | 2.4 | 1.9 | 2.9 | 8.4 |

6

## 0 028 162

The device is very inefficient from the point of view of pulling apart the capsule and the airflow leaves 90% of the powder blend in the capsule unelss the powder is deliberately tipped onto the floor of the chamber. This results in a very low respirable fraction (<3%). The respirable fraction is low (8%) even when the powder is split deliberately out of the capsule before inhalation commences.

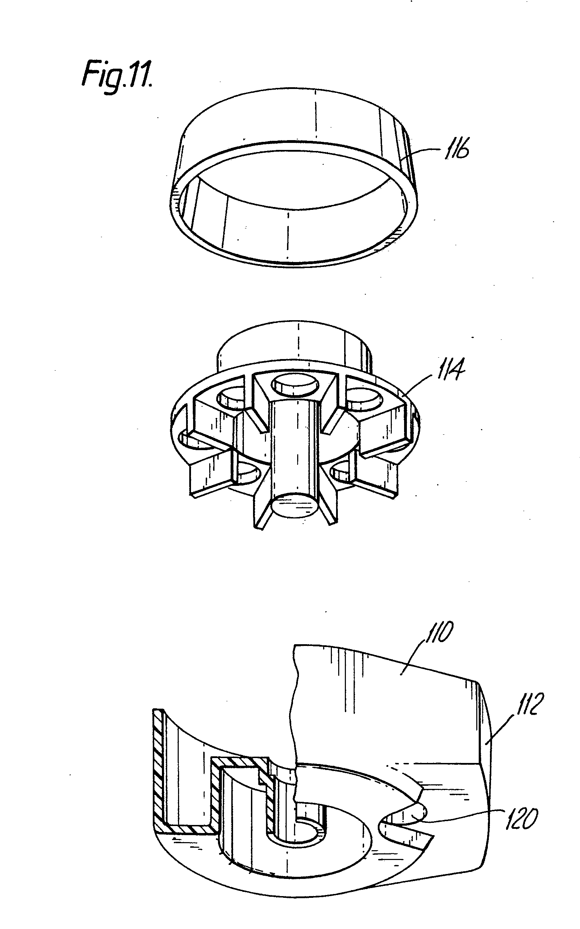

The bronchodilator effect of 200 $\mu$g isoprenaline sulphate as delivered to a sample of 12 patients by the non-propellant device of the invention and by a pressurized aerosol commercially available from Riker Laboratories under the trade mark Medihaler, was compared together with a control in which a placebo preparation was administered by both devices. The bronchodilator effect was compared by measuring the forced expiratory volume in one second ($FEV_1$) for patients before and after administration of the drug and the average results are recorded in Figure 11 as a plot of $FEV_1$ against time.

There was no statistically significant difference between the values of the measured pulmonary function parameters resulting from the application of the drug by each device. However, both modes of application of the drug produced a significant change in pulmonary function compared with a placebo administered by both modes of delivery.

In the embodiment shown in Figure 4, the device is identical to that shown in Figure 1 with the exception that the cylindrical member 2 is elongated to form a skirt portion 40 which completely surrounds the lower piston 8 when in its lower position A thereby protecting the piston from damage and preventing the user's fingers from interfering with its free movement. The skirt portion 40 includes a vertical slot 42 through which arm 44 attached to the piston 8 protrudes thus enabling the piston to be raised and held at its upper position B when required for opening the capsule.

In a second modification of the device of Fig. 1 illustrated in Figure 4a the bottom sliding piston is replaced by a fixed base 13 housing a clutch mechanism 12 at a position corresponding to the higher position B of the sliding piston (Figure 1). This modification makes the device simpler and more compact. In addition, by incorporating a locking mechanism 15 into the device which will enable the piston 6 to be locked securely at its lowest extent of travel with its end surface touching the fixed base, the device may be pre-loaded with a capsule which will be firmly held at all times by the two clutch mechanisms. When medication is then required the user need only unlock the piston and return it to its upper position thus opening the pre-loaded capsule. The piston need not be spring-loaded but may instead be secured at both the lower and higher positions. Pre-loading the device in this manner reduces to a minimum the effort required by the user at a vital time when relief from, for example, an asthmatic attack is being sought.

Figures 5 to 7 show a preferred embodiment of the invention in which the device contains a plurality of capsules which may be used sequentially. The device comprises a body portion 50 having a hinged lid 52, a rotatable carousel 54, a base cap 56 and a movable piston assembly 58. The carousel 54 is formed with a number of apertures 60 adapted to support capsules 62. The capsules 62 comprise a lid component 62A which is a force fit within a base component 62B. The apertures 60 in the carousel have a lip 64 against which the edge of the base component 62B abuts to prevent the base component of the capsule passing completely through the aperture.

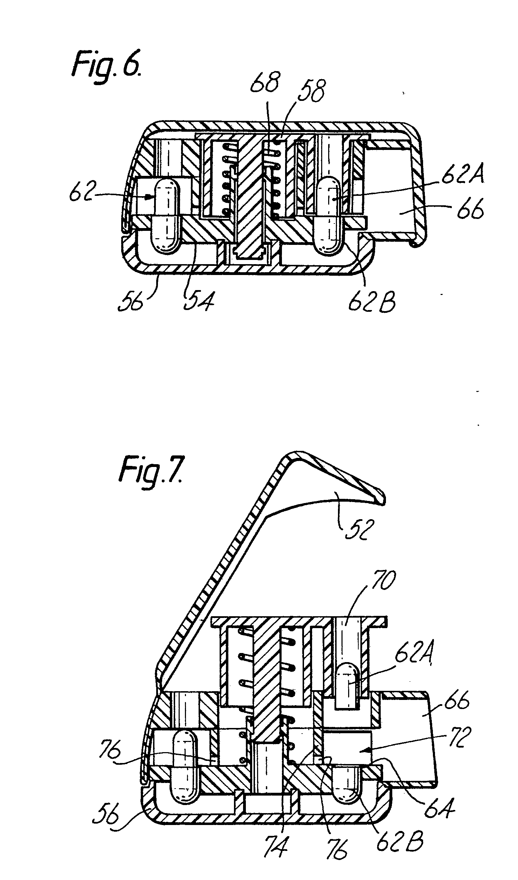

The body of the device includes a short mouthpiece 66 which is covered by the lid 52 when the lid is closed. The piston assembly 58 is movable between the two positions shown in Figures 6 and 7, the assembly being biased by spring 68 so that upon opening of the lid it adopts its raised position as shown in Figure 7. The piston assembly includes an aperture 70 of a suitable size to accommodate the lid component 62A of a capsule 62. The apertures 60 in the carousel and aperture 70 in the piston assembly may include a suitable clutch means as illustrated in Figures 1 to 4.

The chamber 72 of the device is defined by various portions of the carousel, body and piston assembly. In particular, moulded portions 74 on the carousel define the side walls, these mouldings being provided with apertures 76 forming the air inlet ports. The inlet ports are positioned so that in use air flows through the inlet ports directly over the base component 62B of the capsule and out through the mouthpiece 66.

The capsule is opened in the chamber by depressing piston assembly 58 so that the aperture 70 accommodates the lid component of the capsule 62A. Upon releasing the piston assembly it is urged to its raised position as shown in Figure 7 taking the lid component of the capsule 62A with it, thereby opening the capsule as shown in Figure 7. The device is then ready for inhalation.

The device may readily be arranged such that upon opening the lid 52 a capsule in the chamber is automatically opened and the device ready for immediate use. After use the rotatable carousel may be advanced so that a new capsule is positioned in the chamber so that when the device is closed the piston assembly will engage the lid component of the new capsule ready for opening when the lid 52 of the device is opened. Automatic advancement of the carousel may be effected by a suitable ratchet mechanism, which may be operated, for example, by closing the lid.

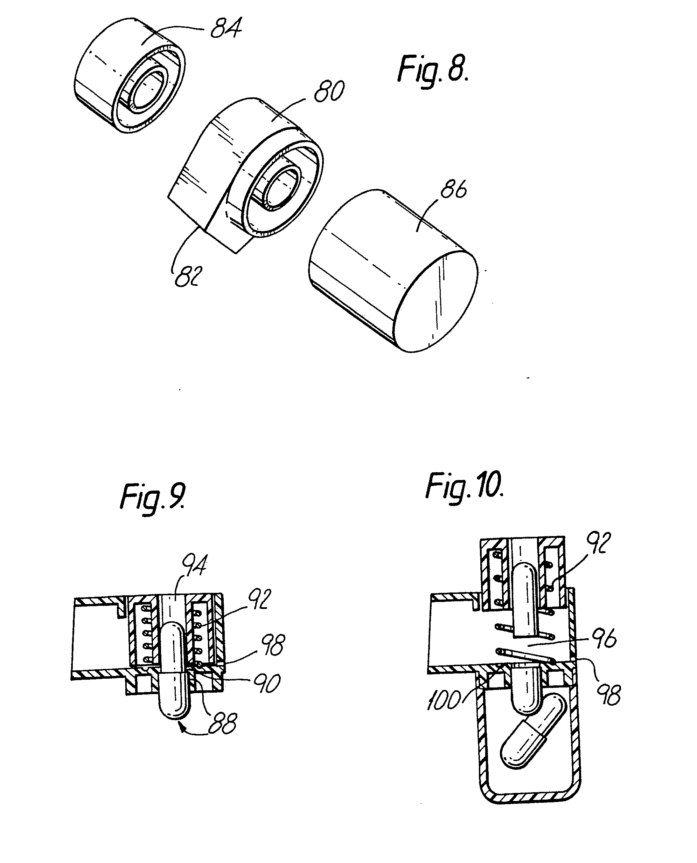

Figures 8 to 10 show an alternative device which must be recharged manually after use. The device comprises a body portion 80 incorporating a mouthpiece 82, a piston assembly 84 and a capsule holder 86. A capsule 62 is manually inserted in an aperture 88 in the body portion as shown in Figure 9. The aperture 88 has a lip portion 90 equivalent to the lip 64 in Figures 5 to 7. The piston assembly 84 is movable between a lower and upper position as shown in Figures 9 and 10

7

respectively, the piston being spring biased by a spring 92. The piston assembly includes an aperture 94 for accommodating the lid component of the capsule 62A in a similar manner to aperture 70 in Figures 5 to 7.

The chamber 96 of the device is defined by the body portion and piston assembly and includes apertures 98 which are arranged so that in use the airflow is directly across the base component of the capsule 62B. The mode of operation of the piston assembly is similar to that shown in Figures 5 to 7 although the piston is depressed manually. The foot of the spring 92 is arranged in an annular depression 100 in the base of the chamber so that there is no disruption of the airflow over the base component of the capsule 62B, the pitch of the spring being sufficiently large so that the coils of the spring do not obstruct the airflow.

The capsule holder 86 may contain a plurality of capsules and is removed from the body portion when a new capsule is to be inserted.

Figures 11 to 13 show a further device according to the invention which comprises a body portion 110 (shown in part section in Figure 11) incorporating a mouthpiece 112, a carousel 114 and a cover 116. In this device the rotatable carousel 114 also forms the piston assembly and the capsules 62 are loaded by inserting their lid component 62A into the apertures of the carousel. The arrangement of the carousel is similar to that shown in Figures 5 to 7 and includes moulded portions to define parts of the side wall of the chamber.

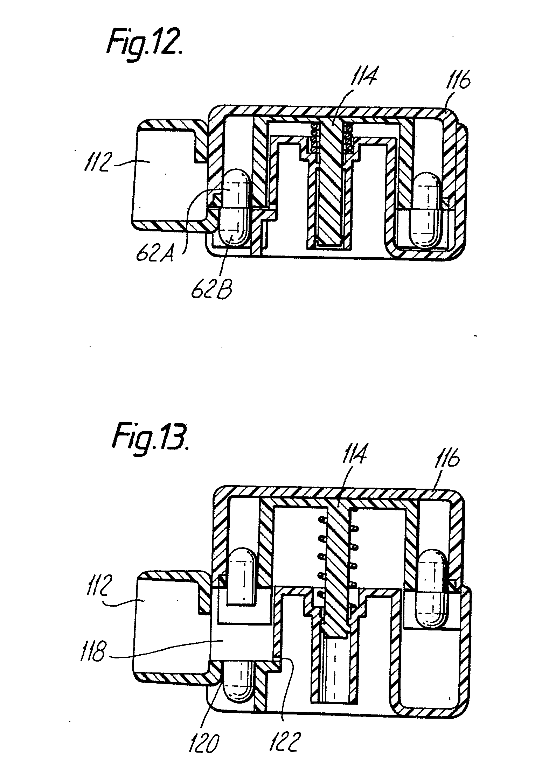

The body portion is moulded to define a substantial portion of the chamber 118 and includes an aperture 120 adapted to accommodate and retain the base component of a capsule 62B. Apertures 122 form the air inlet ports which are arranged so that in use an airflow is established directly across the base component of the capsule. The capsule is opened in the chamber by depressing the cover 116 and hence the carousel 114 so that the base component of the capsule held in the carousel engages in an aperture 120. Upon releasing the cover the carousel and cover is spring biased to the raised position shown in Figure 13 thereby removing the lid component of the capsule 62B from the base component of the capsule 62A. The device is then ready for use. After use the carousel is advanced to position the next capsule within the chamber. The spent base component of the capsule 62B is pushed through the aperture 120 when a new capsule is inserted.

Whilst the arrangement shown in Figures 11 to 13 requires the carousel to be rotated manually, the device may be constructed so that the carousel is advanced automatically, for example by a ratchet mechanism, as the lid is depressed to facilitate ease of operation of the device.

## Claims

1. A breath actuated device for oral inhalation of medicament in finely divided powder form in a capsule comprising a base component and a lid component, the base component being capable of holding said powdered medicament and the lid component being capable of being removed to expose the powdered medicament in the base component, said device comprising a chamber (72) having one or more inlet ports (76) for entry of air and an outlet port in direct communication with a mouthpiece (66) and positioned within the chamber, the base component (62B) of a capsule containing said medicament, characterised in that the base component (62B) of the capsule is mounted substantially vertically and has a substantially horizontal rim defining an aperture and that the inlet ports and outlet port are arranged such that when the device is operated by suction at the mouthpiece a substantially horizontal airflow is established from the inlet ports to the outlet port directly across and substantially parallel to said rim causing particles of medicament to become entrained in said airflow.

2. A device as claimed in Claim 1, characterised in that there is positioned within the chamber means (64, 58) for separating the lid and base components of a capsule containing the medicament whilst holding the capsule substantially vertically to expose an aperture in the base component facilitating access to the powdered medicament.

3. A device as claimed in Claim 1 or Claim 2, characterised in that the base component is positioned so that the rim is at substantially the same height as or lower than the lower extent of the inlet ports.

4. A device as claimed in any one of Claims 1 to 3, characterised in that the lower extent of the inlet port is higher than the lower extent of the outlet port.

5. A device as claimed in Claim 4, characterised in that the vertical extent of the inlet ports is wholly within the vertical extent of the outlet port.

6. A device as claimed in Claim 2 or any claim appendant thereto to 5, characterised in that the means for separating the two components of the capsule comprises an opening means (58) movable between an engaging position in which said opening means engages the lid component (62A) of the capsule held by its base component (62B) in a substantially vertical position, and a dispensing position whereby movement of said opening means from its engaging position to its dispensing position causes separation of the lid component from the base component of the capsule.

7. A device as claimed in Claim 6, characterised in that the opening means comprises a piston (58), spring biased (68) to its dispensing position.

8. A device as claimed in Claim 6 or Claim 7, characterised in that the opening means (58) includes a clutch assembly for gripping the lid of the capsule.

9. A device as claimed in any one of Claims 1 to 8, characterised in that the base of the capsule is mounted by supporting means comprising a piston assembly (8) having a face (13) defining the base of the chamber and including a clutch assembly (12) for gripping the base of the capsule, the piston being urged upwards when the device is operated until it abuts a stop (9) to give impetus to powdered medicament contained in said base of the capsule.

10. A device as claimed in Claim 8 or Claim 9, characterised in that the clutch assembly (12) comprises an annulus of thin flexible material having a plurality of radial slots in the inner circumference to form a ring of teeth which are capable of gripping and retaining a capsule half.

11. A device as claimed in any one of Claims 1 to 8, characterised in that the base of the capsule is mounted by supporting means comprising a carousel (54) supporting a plurality of capsules (62), the carousel being rotatable so that each capsule in turn may be positioned within the chamber, opened and the medicament entrained in said air flow.

12. A device as claimed in Claim 11, characterised in that the base of each of said plurality of capsules is integrally formed in the carousel.

13. A device as claimed in Claim 11 or Claim 12, characterised in that the carousel (54) is constructed to form at least part of the walls (74) and floor of the chamber around each capsule.

14. A device as claimed in any one of Claims 11 to 13 as appendant to Claim 6 or 7, characterised in that the device comprises a body portion having the mouthpiece (66) integral therewith, the body containing and supporting the carousel (54) and a lid (52) hinged to the body which when closed covers the carousel, opening means and mouthpiece.

15. A device as claimed in Claim 14, characterised in that the device is constructed and arranged so that when the lid is closed the opening means is in its engaging position and when the lid is raised the opening means moves to its dispensing position.

16. A device as claimed in any one of Claims 1 to 5, characterised in that the device further comprises a rotatable carousel (114) having a plurality of clutch means for vertically supporting a plurality of capsules by their lid components (62A) with their base components (62B) directed downwardly, the carousel defining a portion of the roof of the chamber (118) so that each capsule may be sequentially introduced into the chamber by rotation of the carousel, the carousel being vertically movable between a raised position and an engaging position in which the base component of a capsule supported in the carousel engages clutch means (120) in the floor of the chamber, whereby movement of the carousel from its engaging position to its raised position causes separation of the lid (62A) from the base component (62B) of the capsule.

**Patentansprüche**

1. Durch Atemluft betätigte Vorrichtung zum oralen Inhalieren eines Medikaments in Form eines feinen Pulvers in einer Kapsel, die ein Unterteil und ein Deckelteil aufweist, wobei das Unterteil das pulverförmige Medikament aufnimmt und das Deckelteil abnehmbar ist, um das pulverförmige Medikament in dem Unterteil freizulegen, wobei die Vorrichtung eine Kammer (72) mit mindestens einer Einlaßöffnung (76) zum Einlaß von Luft und mit einer in direkter Verbindung mit einem Mundstück (76) stehenden Auslaßöffnung aufweist und wobei innerhalb der Kammer das Unterteil (62B) einer das Medikament enthaltenden Kapsel angeordnet ist, dadurch gekennzeichnet, daß das Unterteil (62B) der Kapsel im wesentlichen vertikal angeordnet ist und einen eine Öffnung bildenden, im wesentlichen horizontalen Rand aufweist, und daß die Einlaßöffnungen und die Auslaßöffnung derart angeordnet sind, daß bei Betätigung der Vorrichtung durch Saugen am Mundstück sich eine im wesentlichen horizontale Luftströmung von den Einlaßöffnungen zu der Auslaßöffnung direkt über und im wesentlichen parallel zu dem Rand ausbildet, so daß die Medikamentpartikel in den Luftstrom aufgenommen werden.

2. Vorrichtung nach Anspruch 1, dadurch gekennzeichnet, daß innerhalb der Kammer eine Einrichtung (64, 58) vorgesehen ist, um das Deckelteil und das Unterteil einer das Medikament enthaltenden Kapsel voneinander zu trennen und dabei die Kapsel im wesentlichen vertikal zu halten, um eine Öffnung im Unterteil freizulegen und den Zugang zu den pulverförmigen Medikamenten zu erleichtern.

3. Vorrichtung nach Anspruch 1 oder 2, dadurch gekennzeichnet, daß das Unterteil derart angeordnet ist, daß der Rand im wesentlichen in der gleichen Höhe wie oder niedriger als die niedrigere Erstreckung der Einlaßöffnungen ist.

4. Vorrichtung nach einem der Ansprüche 1—3, dadurch gekennzeichnet, daß die untere Erstreckung der Einlaßöffnung höher liegt als die untere Erstreckung der Auslaßöffnung.

5. Vorrichtung nach Anspruch 4, dadurch gekennzeichnet, daß die Vertikalerstreckung der Einlaßöffnungen vollständig innerhalb der Vertikalerstreckung der Auslaßöffnung ist.

6. Vorrichtung nach Anspruch 2 oder einem von diesem abhängigen Anspruch bis Anspruch 5, dadurch gekennzeichnet, daß die Einrichtung zum Trennen der beiden Kapselteile eine Öffnungsvorrichtung (58) aufweist, die zwischen einer Eingriffsposition, in der die Öffnungsvorrichtung das Deckelteil (62A) der Kapsel ergreift, die mit ihrem Unterteil (62B) in einer im wesentlichen vertikalen Position

9

gehalten wird, und einer Abgabeposition bewegbar ist, so daß die Bewegung der Offnungsvorrichtung aus ihrer Eingriffsposition in ihre Abgabeposition eine Abtrennung des Deckelteils vom Unterteil der Kapsel bewirkt.

7. Vorrichtung nach Anspruch 6, dadurch gekennzeichnet, daß die Öffnungsvorrichtung einen Kolben (58) aufweist, der durch Federvorspannung (68) in die Abgabeposition gedrückt wird.

8. Vorrichtung nach Anspruch 6 oder 7, dadurch gekennzeichnet, daß die Öffnungsvorrichtung (58) eine Kupplungsanordnung zum Ergreifen des Kapseldeckels aufweist.

9. Vorrichtung nach einem der Ansprüche 1 bis 8, dadurch gekennzeichnet, daß das Unterteil der Kapsel durch einen Träger gehalten wird, der eine Kolbenanordnung (8) mit einer Fläche (13), die den Kammerboden bildet, und eine Kupplungsanordnung (12) zum Ergreifen des Kapselunterteils aufweist, und daß der Kolben bei Betätigung der Vorrichtung nach oben gedrückt wird bis er an einem Anschlag (9) anliegt, um das in dem Kapselunterteil enthaltene, pulverförmige Medikament zu fördern.

10. Vorrichtung nach Anspruch 8 oder 9, dadurch gekennzeichnet, daß die Kupplungsanordnung (12) einen Ring aus dünnem, flexiblen Material mit mehreren radialen Schlitzen am Innenumfang zur Ausbildung eines Zahnrings aufweist, durch den eine Kapselhälfte ergriffen und gehalten wird.

11. Vorrichtung nach einem der Ansprüche 1 bis 8, dadurch gekennzeichnet, daß das Kapselunterteil durch einen Träger gehaltert wird, der ein mehrere Kapseln (62) aufnehmendes Karussell (54) aufweist, das derart drehbar ist, daß jede Kapsel innerhalb der Kammer positioniert und geöffnet und das Medikament in den Luftstrom eingesaugt wird.

12. Vorrichtung nach Anspruch 11, dadurch gekennzeichnet, daß das Unterteil jeder Kapsel in dem Karussell einstückig ausgebildet ist.

13. Vorrichtung nach Anspruch 11 oder 12, dadurch gekennzeichnet, daß das Karussell (54) zumindest einen Teil der Wände (74) und des Bodens der Kammer um jede Kapsel bildet.

14. Vorrichtung nach einem der Ansprüche 11 bis 13 in Abhängigkeit von den Ansprüchen 6 oder 7, gekennzeichnet durch einen mit dem Mundstück (66) einstückigen Körperabschnitt, der das Karussell (54) aufnimmt und trägt, und durch einen am Körperabschnitt angelenkten Deckel (52), der im geschlossenen Zustand das Karussell, die Öffnungsvorrichtung und das Mundstück abdeckt.

15. Vorrichtung nach Anspruch 14, dadurch gekennzeichnet, daß bei geschlossenem Deckel die Öffnungsvorrichtung in ihrer Eingriffsposition ist und daß sich beim Anheben des Deckels die Öffnungsvorrichtung in ihre Abgabeposition bewegt.

16. Vorrichtung nach einem der Ansprüche 1 bis 5, dadurch gekennzeichnet, daß die Vorrichtung ferner ein drehbares Karussell (114) mit mehreren Kupplungsanordnungen zum vertikalen Haltern mehrerer Kapseln durch ihre Deckelteile (62A) aufweist, wobei ihre Unterteile (62B) nach unten gerichtet sind, daß das Karussell einen Dachabschnitt der Kammer (118) bildet, so daß beim Drehen das Karussells jede Kapsel sequenziell in die Kammer eingeführt wird, daß das Karussell zwischen einer Hubstellung und einer Eingriffsstellung vertikal bewegbar ist, in der das im Karussell gehalterte Unterteil mit einer Kupplungsanordnung (120) im Kammerboden in Eingriff steht, so daß die Bewegung des Karussells aus seiner Eingriffsstellung in seine Hubstellung den Deckel (62A) vom Unterteil (62B) der Kapsel löst.

**Revendications**

1. Dispositif actionné par la respiration, pour l'inhalation orale d'un médicament sous forme de poudre finement divisée contenu dans une capsule comprenant un élément de base et un élément de couvercle, l'élément de base pouvant contenir le médicament en poudre et l'élément de couvercle pouvant être retiré pour donner accès au médicament en poudre placé dans l'élément de base, ce dispositif comprenant une chambre (72) qui comporte un ou plusieurs orifices d'entrée (76) pour l'admission d'air et un orifice de sortie en communication directe avec l'embouchure (66) et, situé dans la chambre, l'élément de base (62B) d'une capsule contenant le médicament, caractérisé en ce que l'élément de base (62B) de la capsule est monté sensiblement verticalement et comporte un bord sensiblement horizontal définissant une ouverture, et en ce que les orifices d'entrée et l'orifice de sortie sont disposés de sorte que, lorsque le dispositif est actionné par aspiration à l'embouchure, un flux d'air sensiblement horizontal s'établit des orifices d'entrée à l'orifice de sortie, directement transversalement et sensiblement parallèlement au dit bord, ce qui provoque l'entraînement de particules de médicament dans ce flux d'air.

2. Dispositif suivant la revendication 1, caractérisé en ce que des moyens (64, 58) sont situés dans la chambre, pour séparer les éléments de couvercle et de base d'une capsule contenant le médicament, tout en maintenant la capsule sensiblement verticalement, de manière à découvrir une ouverture dans l'élément de base pour faciliter l'accès au médicament en poudre.

3. Dispositif suivant la revendication 1 ou la revendication 2, caractérisé en ce que l'élément de base est situé de manière à ce que le bord soit sensiblement au même niveau ou plus bas que la limite inférieure des orifices d'entrée.

4. Dispositif suivant l'une quelconque des revendications 1 à 3, caractérisé en ce que la limite inférieure de l'orifice d'entrée est plus haute que la limite inférieure de l'orifice de sortie.

5. Dispositif suivant la revendication 4, caractérisé en ce que l'étendue verticale des orifices d'entrée se trouve entièrement à l'intérieur de l'étendue verticale de l'orifice de sortie.

6. Dispositif suivant la revendication 2, ou l'une des revendications qui en dépendent, caractérisé en ce que les moyens de séparation des deux éléments de la capsule comprennent des moyens d'ouverture (58) mobiles entre une position d'engagement, dans laquelle les moyens d'ouverture viennent en prise avec l'élément de couvercle (62A) de la capsule maintenue par son élément de base (62B) dans une position sensiblement verticale, et une position de distribution, le mouvement des moyens d'ouverture de leur position d'engagement à leur position de distribution entraînant la séparation de l'élément de couvercle de l'élément de base de la capsule.

7. Dispositif suivant la revendication 6, caractérisé en ce que les moyens d'ouverture comprennent un piston (58) rappelé par ressort (68) à sa position de distribution.

8. Dispositif suivant la revendication 6 ou la revendication 7, caractérisé en ce que les moyens d'ouverture (58) comportent un dispositif de retenue pour saisir le couvercle de la capsule.

9. Dispositif suivant l'une quelconque des revendications 1 à 8, caractérisé en ce que la base de la capsule est tenue par des moyens de maintien comprenant un piston (8), dont une face (13) définit la base de la chambre et comporte un dispositif de retenue (12) pour saisir la base de la capsule, le piston étant rappelé vers le haut, lorsque le dispositif est utilisé, jusqu'à ce qu'il rencontre une butée (9) de manière à donner une impulsion au médicament en poudre contenu dans la base de la capsule.

10. Dispositif suivant la revendication 8 ou la revendication 9, caractérisé en ce que les moyens de retenue (12) comprennent un anneau de matière flexible mince comportant dans sa circonférence intérieure une pluralité de fentes radiales, de manière à constituer une denture annulaire capable de saisir ou de retenir une moitié de capsule.

11. Dispositif suivant l'une quelconque des revendications 1 à 8, caractérisé en ce que la base de la capsule est montée dans des moyens de maintien comprenant un carrousel (54) qui supporte une pluralité de capsules (62), le carrousel pouvant tourner de sorte que chaque capsule successivement peut être placée dans la chambre, ouverte et le médicament entraîné dans le flux d'air.

12. Dispositif suivant la revendication 11, caractérisé en ce que la base de chaque capsule de ladite pluralité de capsules est formée solidairement dans le carrousel.

13. Dispositif suivant la revendication 11 ou la revendication 12, caractérisé en ce que le carrousel (54) est construit de manière à constituer au moins une partie des parois (74) et du plancher de la chambre autour de chaque capsule.

14. Dispositif suivant l'une quelconque des revendications 11 à 13, en ce qu'elles dépendent de la revendication 6 ou 7, caractérisé en ce que le dispositif comprend un corps comportant l'embouchure (66) solidairement du corps, le corps contenant et supportant le carrousel (54), et un couvercle (52) articulé sur le corps et qui, lorsqu'il est fermé, recouvre le carrousel, les moyens d'ouverture et l'embouchure.

15. Dispositif suivant la revendication 14, caractérisé en ce que le dispositif est construit et agencé de sorte que, lorsque le couvercle est fermé, les moyens d'ouverture sont dans leur position d'engagement et, lorsque le couvercle est relevé, les moyens d'ouverture se déplacent à leur position de distribution.

16. Dispositif suivant l'une quelconque des revendications 1 à 5, caractérisé en ce que le dispositif comprend également un carrousel tournant (114) comportant une pluralité de moyens de retenue pour supporter verticalement une pluralité de capsules par leurs éléments de couvercle (62A), avec leurs éléments de base (62B) dirigés vers le bas, le carrousel définissant une partie du toît de la chambre (118) de sorte que chaque capsule peut être successivement introduite dans la chambre par rotation du carrousel, le carrousel étant mobile verticalement entre une position relevée et une position d'engagement dans laquelle l'élément de base d'une capsule supportée dans le carrousel vient en prise avec des moyens de retenue (120) dans le plancher de la chambre, de sorte que le mouvement du carrousel de sa position d'engagement à sa position relevée provoque la séparation du couvercle (62A) de l'élément de base (62B) de la capsule.

11

0 028 162

Fig.1.

Fig.2.

Fig.3.

1

# Fig. 4.

Fig.4a.

*Fig.5.*

*Fig. 6.*

68  58

62

62A

66

56  54  62B

*Fig. 7.*

52

70

62A

66

76

72

56

74  76  62B

64

Fig. 8.

Fig. 9.

Fig. 10.

*Fig.11.*

116

114

110

112

120

*Fig.12.*

114   116

112

62A

62B

*Fig.13.*

114   116

112

118

120   122

Fig.14.

0028162